# EUROPEAN PATENT SPECIFICATION

(11) **EP 4 438 667 B1**
(45) Date of publication and mention of the grant of the patent: **28.05.2025**
(21) Application number: 23193904.2
(22) Date of filing: 29.08.2023
(51) Int. Cl.: C08L 1/02, A61K 8/73, C08L 1/12

(54) **CELLULOSE PARTICLE**
CELLULOSEPARTIKEL
PARTICULE DE CELLULOSE

(30) Priority: 28.03.2023 JP 2023052437
(43) Date of publication of application: 02.10.2024
(73) Proprietor: FUJIFILM Business Innovation Corp., Minato-ku Tokyo (JP)
(72) Inventor: ISHIZUKA, Takahiro, Minamiashigara-shi, Kanagawa (JP); OKI, Masahiro, Minamiashigara-shi, Kanagawa (JP); MATOBA, Shota, Minamiashigara-shi, Kanagawa (JP); YAO, Kenji, Minamiashigara-shi, Kanagawa (JP); TAGUCHI, Tetsuya, Minamiashigara-shi, Kanagawa (JP); HAMANO, Hirokazu, Minamiashigara-shi, Kanagawa (JP)
(74) Representative: Kurig, Thomas

(56) References cited:
- EP-A1- 3 932 974
- WO-A1-2022/137679

## Description

### Background

### (i) Technical Field

The present disclosure relates to a cellulose particle.

### (ii) Related Art

Japanese Patent No. 6921293 discloses "resin beads produced by surface-treating, with a solid surface treatment agent, core beads made of a resin containing cellulose as a main component, wherein the resin beads have a particle diameter of 50 µm or less at a cumulative volume of 50%, a sphericity of 0.7 to 1.0, a surface smoothness of 70% to 100%, and a crystallinity of 60% or less."

Japanese Patent No. 6212424 discloses "a surface-treated cellulose including cellulose particles each having a surface represented by a specific chemical formula."

WO 2022/137679 A1 discloses cellulose particles coated with magnesium stearate.

EP 3 932 974 A1 discloses cellulose particles coated with a first coating layer of cationic polyalkyleneimine, polyallylamine or polyvinylamine, and a second coating layer of a hydrophobic compound such as a fatty acid.

### Summary

Accordingly, it is an object of the present disclosure to provide a cellulose particle having a base particle containing cellulose as a main component, wherein the cellulose particle shows more rapid biodegradation, has better water resistance, and keeps water resistance even after being stored in water compared with cellulose particles each having a first coating layer disposed on the surface of the base particles and containing only a neutral polysaccharide and a second coating layer disposed on the first coating layer and containing a hydrophobic compound.

According to a first aspect of the present disclosure, there is provided a cellulose particle including: a base particle containing cellulose as a main component; a first coating layer disposed on a surface of the base particle and containing a multivalent metal salt of an acid functional group-containing resin; and a second coating layer disposed on the first coating layer and containing at least one hydrophobic compound selected from fatty acids, multivalent metal salts of fatty acids, amino acid compounds, and multivalent metal salts of amino acid compounds.

According to a second aspect of the present disclosure, there is provided the cellulose particle according to the first aspect wherein the multivalent metal salt of the acid functional group-containing resin is a multivalent metal salt of a polysaccharide.

According to a third aspect of the present disclosure, there is provided the cellulose particle according to the first or second aspect wherein the multivalent metal salt of the acid functional group-containing resin has at least one multivalent metal selected from Ca and Al.

According to a fourth aspect of the present disclosure, there is provided the cellulose particle according to any one of the first to third aspects wherein a ratio of the multivalent metal to the resin in the multivalent metal salt of the acid functional group-containing resin is 1/4 or more and 4/1 or less in terms of molar ratio.

According to a fifth aspect of the present disclosure, there is provided the cellulose particle according to the fourth aspect wherein a ratio of the multivalent metal to the resin in the multivalent metal salt of the acid functional group-containing resin is 1/3 or more and 3/1 or less in terms of molar ratio.

According to a sixth aspect of the present disclosure, there is provided the cellulose particle according to any one of the first to fifth aspects wherein the hydrophobic compound is at least one hydrophobic compound selected from the multivalent metal salts of fatty acids and the multivalent metal salts of amino acid compounds.

According to a seventh aspect of the present disclosure, there is provided the cellulose particle according to the sixth aspect wherein the multivalent metal salts of fatty acids and the multivalent metal salts of amino acid compounds have at least one multivalent metal selected from Ca and Al.

According to an eighth aspect of the present disclosure, there is provided the cellulose particle according to any one of the first to seventh aspects wherein the multivalent metal salt of the acid functional group-containing resin is a multivalent metal salt of a polysaccharide,
the multivalent metal salt of the acid functional group-containing resin has at least one multivalent metal selected from Ca and Al, and
a ratio of the multivalent metal to the resin in the multivalent metal salt of the acid functional group-containing resin is 1/3 or more and 3/1 or less in terms of molar ratio.

According to a ninth aspect of the present disclosure, there is provided the cellulose particle according to the eighth aspect wherein the hydrophobic compound is at least one hydrophobic compound selected from the multivalent metal salts of fatty acids and the multivalent metal salts of amino acid compounds.

According to a tenth aspect of the present disclosure, there is provided the cellulose particle according to the ninth aspect wherein the multivalent metal salts of fatty acids and the multivalent metal salts of amino acid compounds have at least one multivalent metal selected from Ca and Al.

According to the first aspect of the present disclosure, the cellulose particle having the base particle containing cellulose as a main component shows more rapid biodegradation, has better water resistance, and keeps water resistance even after being stored in water compared with a cellulose particle having a first coating layer disposed on the surface of a base particle and containing only a neutral polysaccharide and a second coating layer disposed on the first coating layer and containing a hydrophobic compound.

According to the second aspect of the present disclosure, the cellulose particle shows more rapid biodegradation, has better water resistance, and keeps water resistance even after being stored in water compared with a particle in which the multivalent metal salt of an acid functional group-containing resin is a multivalent metal salt of a synthetic resin.

According to the third aspect of the present disclosure, the cellulose particle shows more rapid biodegradation, has better water resistance, and keeps water resistance even after being stored in water compared with a particle in which the multivalent metal salt of an acid functional group-containing resin has a multivalent metal other than Ca and Al.

According to the fourth aspect of the present disclosure, the cellulose particle shows more rapid biodegradation, has better water resistance, and keeps water resistance even after being stored in water compared with a particle in which the ratio of the multivalent metal to the resin in the multivalent metal salt of the acid functional group-containing resin is less than 1/4 or more than 4/1 in terms of molar ratio.

According to the fifth aspect of the present disclosure, the cellulose particle shows more rapid biodegradation, has better water resistance, and keeps water resistance even after being stored in water compared with a particle in which the ratio of the multivalent metal to the resin in the multivalent metal salt of the acid functional group-containing resin is less than 1/3 or more than 3/1 in terms of molar ratio.

According to the sixth aspect of the present disclosure, the cellulose particle shows more rapid biodegradation, has better water resistance, and keeps water resistance even after being stored in water compared with a particle in which the hydrophobic compound is at least one hydrophobic compound selected from fatty acids and amino acid compounds.

According to the seventh aspect of the present disclosure, the cellulose particle shows more rapid biodegradation, has better water resistance, and keeps water resistance even after being stored in water compared with a particle in which the multivalent metal salts of fatty acids and the multivalent metal salts of amino acid compounds have a multivalent metal other than Ca and Al.

According to the eighth aspect of the present disclosure, the cellulose particle shows more rapid biodegradation, has better water resistance, and keeps water resistance even after being stored in water compared with a particle in which the multivalent metal salt of an acid functional group-containing resin is a multivalent metal salt of a synthetic resin, the multivalent metal salt of an acid functional group-containing resin has a multivalent metal other than Ca and Al, or the ratio of the multivalent metal to the resin in the multivalent metal salt of the acid functional group-containing resin is less than 1/4 or more than 4/1 in terms of molar ratio.

According to the ninth aspect of the present disclosure, the cellulose particle shows more rapid biodegradation, has better water resistance, and keeps water resistance even after being stored in water compared with a particle in which the hydrophobic compound is at least one hydrophobic compound selected from fatty acids and amino acid compounds.

According to the tenth aspect of the present disclosure, the cellulose particle shows more rapid biodegradation, has better water resistance, and keeps water resistance even after being stored in water compared with a particle in which the multivalent metal salts of fatty acids and the multivalent metal salts of amino acid compounds have a multivalent metal other than Ca and Al.

### Detailed Description

An exemplary embodiment of the present disclosure will be described below. The following description and Examples are for illustrating the exemplary embodiment, and are not intended to limit the scope of the exemplary embodiment.

With regard to value ranges described stepwise in this specification, the upper limit or the lower limit of one value range may be replaced by the upper limit or the lower limit of another value range. The upper limit or lower limit of any value range described in this specification may be replaced by a value described in Examples.

In this specification, the term "step" includes not only an independent step but also a step that cannot be clearly distinguished from other steps but may accomplish an intended purpose.

Each component may include two or more corresponding substances.

The amount of each component refers to, when there are two or more substances corresponding to each component, the total amount of the substances, unless otherwise specified.

The "(meth)acrylic" refers to at least one of acrylic and methacrylic, and the "(meth)acrylate" refers to at least one of acrylate and methacrylate.

### Cellulose Particles

Cellulose particles according to an exemplary embodiment each include:
a base particle (hereinafter also referred to as a "cellulose base particle" containing cellulose as a main component;
a first coating layer disposed on the surface of the base particle and containing a multivalent metal salt of an acid functional group-containing resin; and
a second coating layer disposed on the first coating layer and containing at least one hydrophobic compound selected from fatty acids, multivalent metal salts of fatty acids, amino acid compounds, and multivalent metal salts of amino acid compounds.

Having the above structure, the cellulose particles according to the exemplary embodiment show more rapid biodegradation, have better water resistance, and keep water resistance even after being stored in water. In other words, the cellulose particles according to the exemplary embodiment keep water repellency to hot water and keep a moist texture. The reason for this is assumed as described below.

Cellulose particles are expected as an alternative to resin particles in various fields because of high biodegradability and durability. However, cellulose particles have limited applications because cellulose particles have a rigid molecular structure and strong intermolecular and intramolecular hydrogen bonds.

To address this issue, there are known techniques for improving the texture of cellulose particles by, for example, surface-treating base particles containing cellulose as a main component with a group derived from a dibasic acid having two carboxyl groups, a metallic soap, or other materials (e.g., Japanese Patent No. 6921293, Japanese Patent No. 6212424).

In these techniques, however, the surface treatment layer has low adhesion strength, and the cellulose particles thus have low water resistance. Moreover, the water resistance of the cellulose particles decreases when the cellulose particles are stored in water.

The reason why the water resistance decreases when the cellulose particles are stored in water is that water enters the spaces between the base particles and the surface treatment layer to peel the surface treatment layer so that the surface of the base particles containing cellulose as a main component is exposed to wetting with water.

The water resistance of the cellulose particles according to the exemplary embodiment is improved by disposing, on the base particles containing rapidly biodegradable cellulose as a main component, a second coating layer containing at least one hydrophobic compound selected from fatty acids, multivalent metal salts of fatty acids, amino acid compounds, and multivalent metal salts of amino acid compounds.

A first coating layer containing a multivalent metal salt of an acid functional group-containing resin is interposed between the base particles and the second coating layer. Since the multivalent metal salt of the acid functional group-containing resin forms a physically cross-linked structure, the multivalent metal salt provides the first coating layer with water resistance to hinder water from entering the spaces between the base particles and the second coating layer.

From the above reason, the cellulose particles according to the exemplary embodiment are assumed to show rapid biodegradation, have high water resistance, and keep water resistance even after being stored in water.

Whether the multivalent metal salt of the acid functional group-containing resin forms a physically cross-linked structure is determined by the following method.

A 0.2 mass% aqueous solution of an intended substance is prepared. If the aqueous solution has pH 6.5 or lower, the pH is adjusted to 7.0 or higher and 8.5 or lower by adding an aqueous solution of sodium hydroxide. When the aqueous solution is difficult to flow, the aqueous solution is diluted or heated to make it flow.

A 1 mass% aqueous solution of potash alum is added and mixed such that the amount of potassium alum is 15 mass% with respect to the amount of the intended substance of the aqueous solution. When the mixed solution becomes turbid, increases in viscosity, or is gelled after being left to stand at room temperature (25°C) for 30 minutes, it is determined that the physically cross-linked structure is formed.

The cellulose particles according to the exemplary embodiment will be described below in detail.

### Base Particles

The base particles are particles on which the first coating layer and the second coating layer are to be formed, and the base particles contain cellulose as a main component.

The phrase "containing cellulose as a main component" means that the amount of cellulose with respect to the base particles is 90 mass% or more (95 mass% or more, 98 mass% or more, or 100 mass%).

The number-average molecular weight of cellulose is preferably 37,000 or more and more preferably 45,000 or more.

The upper limit of the number-average molecular weight of cellulose may be, not necessarily, for example, 100,000 or less.

When the number-average molecular weight of cellulose is 37,000 or more, the cellulose particles have higher biodegradability and a better moist texture. The reason for this is assumed as described below.

When the number-average molecular weight is 37,000 or more, there are less terminal hydroxyl groups and thus less hydroxyl groups per unit volume, so that the number and strength of intermolecular and intramolecular hydrogen bonds are reduced. As a result, the cellulose particles have high flexibility and easily increase in moist texture.

The number-average molecular weight of cellulose is measured by gel permeation chromatography (differential refractometer available from Optilab T-rEX/Wyatt Technology, multi-angle light scattering detector available from DAWN HELEOS II/Wyatt Technology, column TSKgel α-M × 1, α-3000 × 1 available from Tosoh Corporation) using dimethylacetamide (0.1 M lithium chloride added) as a solvent.

### Other Components

The base particles may contain other additives.

Examples of other additives include plasticizers, flame retardants, compatibilizers, release agents, light stabilizers, weather stabilizers, colorants, pigments, modifiers, anti-drip agents, antistatic agents, anti-hydrolysis agents, fillers, reinforcing agents (e.g., glass fiber, carbon fiber, talc, clay, mica, glass flakes, milled glass, glass beads, crystalline silica, alumina, silicon nitride, aluminum nitride, boron nitride), acid acceptors for preventing acetic acid release (e.g., oxides, such as magnesium oxide and aluminum oxide; metal hydroxides, such as magnesium hydroxide, calcium hydroxide, aluminum hydroxide, and hydrotalcite; calcium carbonate; talc), and reactive trapping agents (e.g., epoxy compounds, acid anhydride compounds, carbodiimides).

The amount of other components may be 0 mass% or more and 5 mass% or less with respect to the total amount of the base particles. The "0 mass%" means that the base particles do not contain any other components.

### First Coating Layer

The first coating layer is an intermediate layer interposed between the cellulose base particles and the second coating layer.

The first coating layer contains a multivalent metal salt of an acid functional group-containing resin.

Examples of the acid functional group of the acid functional group-containing resin include carboxyl group, sulfo group, sulfonimide group, phosphate group, and phosphonate group. The acid functional group may be carboxyl group among these groups to improve water resistance and keep water resistance.

Examples of the acid functional group-containing resin include cellulose acylates having acid functional groups, polysaccharides having acidic functional groups, and synthetic resins having acid functional groups.

Examples of cellulose acylates include carboxyalkyl celluloses (e.g., carboxymethyl cellulose).

Examples of polysaccharides include polysaccharides having sulfuric acid or phosphoric acid, polysaccharides having uronic acid (e.g., glucuronic acid, iduronic acid, galacturonic acid, mannuronic acid), and polysaccharides having the structures of both of these acids. Specific examples of polysaccharides include hyaluronic acid, gellan gum, deacylated gellan gum (DAG), rhamsan gum, diutan gum, xanthan gum, carrageenan, hexuronic acid, fucoidan, pectin, pectic acid, pectinic acid, heparan sulfate, heparin, heparitin sulfate, keratosulfate, chondroitin sulfate, dermatan sulfate, rhamnan sulfate, and alginic acid.

Examples of synthetic resins include poly(meth)acrylate resins, polymethacrylate resins, and copolymer resins of (meth)acrylates and alkyl (meth)acrylates (e.g., acrylates/C10-30 alkyl acrylate crosspolymer).

Examples of the multivalent metal of the multivalent metal salt of the acid functional group-containing resin include alkaline earth metals (Ca (calcium), Ba (barium), Mg (magnesium)), Al (aluminum), Zn (zinc), Cu (copper), Fe (iron), Zr (zirconium), and Ti (titanium).

To improve water resistance and improve water resistance maintenance, the multivalent metal salt of the acid functional group-containing resin is preferably a multivalent metal salt of a polysaccharide, more preferably at least one salt selected from Ca and Al salts of polysaccharides. This is because these multivalent metal salts of polysaccharides are considered to form a physically cross-linked structure with high water resistance.

To improve water resistance and improve water resistance maintenance, the ratio of the multivalent metal to the resin in the multivalent metal salt of the acid functional group-containing resin is preferably 1/4 or more and 4/1 or less, more preferably 1/3 or more and 3/1 or less in terms of molar ratio.

To improve water resistance and improve water resistance maintenance, the coating weight of the first coating layer on the base particles is preferably 0.1 mass% or more and 10 mass% or less, more preferably 0.2 mass% or more and 8 mass% or less, still more preferably 0.3 mass% or more and 5 mass% or less.

The proportion of the multivalent metal salt of the resin in the entire first coating layer is preferably 90 mass% or more and 100 mass% or less, more preferably 95 mass% or more and 100 mass% or less.

### Second Coating Layer

The second coating layer is disposed on the first coating layer and forms an outermost layer of each cellulose particle.

The second coating layer contains at least one hydrophobic compound selected from fatty acids, multivalent metal salts of fatty acids, amino acid compounds, and multivalent metal salts of amino acid compounds.

### Fatty Acids

Fatty acids are linear or branched, saturated or unsaturated fatty acids. The fatty acids may be mixtures of saturated fatty acids and unsaturated fatty acids.

To improve water resistance and improve water resistance maintenance, the fatty acids may be fatty acids with 16 or more and 22 or less carbon atoms (or 18 or more and 20 or less carbon atoms). Specific examples of linear fatty acids with 16 or more and 22 or less carbon atoms include stearic acid, behenic acid, arachidic acid, and palmitic acid.

Examples of the multivalent metal salts of fatty acids include the multivalent metal salts of the fatty acids described above as examples.

Examples of the multivalent metals of the multivalent metal salts of the fatty acids include alkaline earth metals (Ca (calcium), Ba (barium), Mg (magnesium)), Al (aluminum), Zn (zinc), Cu (copper), Fe (iron), Zr (zirconium), and Ti (titanium).

When the fatty acids have 16 or more carbon atoms in the fatty acids and the multivalent metal salts of the fatty acids, the fatty acid groups are short, and the sponge effect is sufficient. This improves flexibility. When the fatty acids have 22 or less carbon atoms, the repulsion of the fatty acid groups is reduced, and the second coating layer is difficult to peel off.

### Amino Acid Compounds and Multivalent Metal Salts of Amino Acid Compounds

Amino acid compounds refer to amino acids and amino acid derivatives.

Examples of amino acid compounds include myristoyl glutamic acid, lauroyl glutamic acid, stearoyl glutamic acid, lauroyl aspartic acid, dilauramidoglutamide lysine, lauroyl lysine, lauryl arginine, and myristyl leucine.

Examples of the multivalent metal salts of amino acid compounds include the multivalent metal salts of the amino acid compounds described above as examples.

Examples of the multivalent metals of the multivalent metal salts of the amino acid compounds include alkaline earth metals (Ca (calcium), Ba (barium), Mg (magnesium)), Al (aluminum), Zn (zinc), Cu (copper), Fe (iron), Zr (zirconium), and Ti (titanium).

The hydrophobic compounds are preferably multivalent metal salts of fatty acids, or multivalent metal salts of amino acid compounds, more preferably multivalent metal salts of fatty acids, or at least one salt selected from Ca and Al salts of amino acid compounds. This is because these multivalent metal salts of polysaccharides are considered to form a physically cross-linked structure with high water resistance and improve the water resistance of the second coating layer itself.

To improve water resistance and improve water resistance maintenance, the coating weight of the second coating layer on the base particles is preferably 1 mass% or more and 20 mass% or less, more preferably 1 mass% or more and 15 mass% or less, still more preferably 2 mass% or more and 10 mass% or less.

The proportion of the hydrophobic compound in the entire second coating layer is preferably 90 mass% or more and 100 mass% or less, more preferably 95 mass% or more and 100 mass% or less.

To improve water resistance and improve water resistance maintenance, the proportion of the coating weight of the first coating layer to the coating weight of the second coating layer is preferably 0.01 or more and 10 or less, more preferably 0.01 or more and 4 or less, still more preferably 0.05 or more and 2.5 or less.

### External Additives

The cellulose particles according to the exemplary embodiment may have external additives externally added thereto.

The external addition of the external additives prevents or reduces secondary aggregation of the particles so that the particles tend to exhibit their original properties. It is thus easy to improve water resistance and water resistance maintenance. The external addition of the external additives also prevents or reduces decreases in biodegradability.

Examples of the external additives include silicon-containing compound particles, metallic soap particles, fatty acid ester particles, and metal oxide particles.

The silicon-containing compound particles refer to particles containing silicon.

The silicon-containing compound particles may be particles containing only silicon or may be particles containing silicon and other elements.

The silicon-containing compound particles may be silica particles.

The silica particles are particles containing silica, that is SiO₂, as a main component and may be crystalline or amorphous. The silica particles may be particles produced by using a silicon compound, such as water glass or an alkoxysilane, as a raw material, or may be particles made by grinding quartz.

The metallic soap particles are particles containing a metallic soap as a main component.

The particles containing a metallic soap as a main component refer to particles containing 90 mass% or more of the metallic soap with respect to the particles.

Metallic soaps are fatty acid metal salts in which fatty acids are bonded to metals.

Examples of fatty acid metal salts include metal salts of fatty acids with 10 or more and 25 or less (or 12 or more and 22 or less) carbon atoms. Examples of metal salts of fatty acids with 10 or more and 25 or less carbon atoms include metal salts of stearic acid, metal salts of palmitic acid, metal salts of lauric acid, metal salts of oleic acid, metal salts of linoleic acid, and metal salts of ricinoleic acid.

Examples of the metals in the fatty acid metal salts include divalent metals.

Examples of the metals in the fatty acid metal salts include magnesium, calcium, aluminum, barium, and zinc.

The fatty acid ester particles are particles containing fatty acid ester particles as a main component.

The particles containing fatty acid ester particles as a main component refer to particles containing 90 mass% or more of the fatty acid ester particles with respect to the particles.

Examples of fatty acid esters include esters produced by the reactions between saturated fatty acids with 10 or more and 25 or less carbon atoms and alcohols with 10 or more and 25 or less carbon atoms.

Examples of fatty acid esters include stearyl stearate, stearyl laurate, and stearyl palmitate.

The metal oxide particles are particles containing a metal oxide as a main component.

The particles containing a metal oxide as a main component refer to particles containing 90 mass% or more of the metal oxide with respect to the particles.

The metal oxide may be an oxide of a metal other than silicon.

Examples of the metal oxide include zinc oxide, magnesium oxide, iron oxide, and aluminum oxide.

The volume-average particle diameter of the external additives is preferably 1 nm or more and 100 nm or less, more preferably 5 nm or more and 30 nm or less to improve texture (specifically moist texture).

The volume-average particle diameter of the external additives is determined by the same method as for the volume-average particle diameter of the cellulose particles.

The amount of the external additives externally added may be 0.1 mass% or more and 2 mass% or less with respect to the total mass of the cellulose particles (cellulose particles with no external additives externally added).

### Volume-Average Particle Diameter

The volume-average particle diameter of the cellulose particles according to the exemplary embodiment is preferably 3 µm or more and 10 µm or less, more preferably 4 µm or more and 9 µm or less, still more preferably 5 µm or more and 8 µm or less.

When the volume-average particle diameter of the cellulose particles according to the exemplary embodiment is 3 µm or more and 10 µm or less, the particle diameter is appropriate to improve water resistance and water resistance maintenance. The flexibility increases to improve moist texture.

The volume-average particle diameter of the cellulose particles is measured as described below.

The particle diameter is measured by using a LS particle size distribution analyzer "Beckman Coulter LS13 320 available from Beckman Coulter, Inc.), and the volume-based cumulative distribution of the particle diameter is drawn from the smaller particle diameter side, and the particle diameter at a cumulative volume of 50% is determined as a volume-average particle diameter.

### Method for Producing Cellulose Particles

A method for producing the cellulose particles according to the exemplary embodiment is, for example, as described below.

### Step for Producing Cellulose Particles (Base Particles)

(1) First, a cellulose acylate is dissolved in a water-soluble organic solvent A to prepare a cellulose acylate solution A.
(2) Next, the cellulose acylate solution A is added to a calcium carbonate dispersion, which is a dispersion of calcium carbonate in water, and the mixture is stirred to prepare a cellulose acylate solution B.
(3) Next, the cellulose acylate solution B is added to a mixed solution of carboxymethyl cellulose, a water-soluble organic solvent B, and water, and the mixture is stirred at high speed to prepare a cellulose acylate solution C.
(4) Next, sodium hydroxide is added to the cellulose acylate solution C, and a cellulose acylate dispersion C is then heated to remove the water-soluble organic solvents A and B. Hydrochloric acid is added to form cellulose acylate particles. The cellulose acylate particles are then filtered out, and the filtered-out cellulose acylate particles are dispersed in water to prepare a cellulose acylate particle dispersion.
(5) Next, sodium hydroxide is added to the cellulose acylate particle dispersion, and the cellulose acylate particle dispersion is heated in a weak alkaline environment and stirred, whereby the cellulose acylate particles undergo saponification to prepare a cellulose particle suspension.
(6) Next, the pH of the cellulose particle suspension is adjusted to near neutral (e.g., in the range of 6.5 or higher and 7 or lower) by adding hydrochloric acid to the cellulose particle suspension. The cellulose particles are then filtered out and washed with pure water repeatedly. The filtered-out cellulose particles (base particles) are then dried after the conductivity of the filtrate reaches 10 µS/cm or less.

The cellulose acylate is a cellulose derivative in which at least one of hydroxy groups of cellulose is substituted (acylated) with an aliphatic acyl group. Specifically, the cellulose acylate is a cellulose derivative in which at least one of hydroxy groups of cellulose is substituted with -CO-R^{AC} (R^{AC} represents an aliphatic hydrocarbon group).

The water-soluble organic solvent A is a solvent that dissolves 0.1 mass% or more and 10 mass% or less of water with respect to the solvent at 25°C. Examples of the water-soluble organic solvent A include ethyl acetate and butyl acetate.

The water-soluble organic solvent B is a solvent that dissolves 0.1 mass% or more and 10 mass% or less of water with respect to the solvent at 25°C. Examples of the water-soluble organic solvent B include methyl ethyl ketone and acetone.

### Step for Forming Coating Layers

First, an acid functional group-containing resin is added to a slurry of the base particles. If the slurry has pH 6.5 or lower, the slurry is neutralized to pH 7.0 or higher and pH 8.0 or lower by adding an alkaline aqueous solution (e.g., aqueous solution of sodium hydroxide) to the slurry. Subsequently, a multivalent metal salt (e.g., chlorides, such as calcium chloride, magnesium chloride, and aluminum chloride; and sulfates, such as potassium alum) is added to the slurry, and the mixture is stirred. In this process, the multivalent metal salt of the acid functional group-containing resin is cross-linked and adsorbed to the surfaces of the base particles to form a first coating layer composed of the multivalent metal salt of the resin.

Next, a slurry of the base particles having the first coating layer formed thereon is heated, and an aqueous solution containing a hydrophobic compound being a non-metal salt or metal salt is then added separately. When the hydrophobic compound is a fatty acid or an amino acid compound, the slurry is neutralized by adding a sodium hydroxide solution.

Next, a chloride of a multivalent metal or an aqueous solution of hydrochloric acid is added to the slurry to deposit the hydrophobic compound. This forms a second coating layer composed of the hydrophobic compound on the first coating layer.

In the case of adding a chloride of a multivalent metal, a multivalent metal salt of a hydrophobic compound (i.e., multivalent metal salt of fatty acid, multivalent metal salt of amino acid compound) is deposited. In the case of adding an aqueous solution of hydrochloric acid, a hydrophobic compound being a non-metal salt (i.e., fatty acid, amino acid compound) is deposited.

Next, the cellulose particles having the coating layers are taken out of the aqueous solution. The cellulose particles having the coating layers are taken out by, for example, filtering the aqueous solution. The taken-out cellulose particles having the coating layers may be washed with water. The coating layer forming materials not used for coating are removed accordingly. The cellulose particles having the coating layers are dried to form the cellulose particles according to the exemplary embodiment.

### External Addition Step

External additives may be added to the formed cellulose particles.

The external addition step includes, for example, adding external additives to the cellulose particles by using, for example, a mixing mill, a V-type blender, a Henschel mixer, or a Loedige mixer.

### Usage

The cellulose particles according to the exemplary embodiment can be used as, for example, granular materials for cosmetics, rolling agents, abrasives, scrubbing agents, display spacers, bead molding materials, light diffusing particles, resin reinforcing agents, refractive index control agents, biodegradation promoters, fertilizers, water absorbent particles, toner particles, and anti-blocking particles.

The cellulose particles according to the exemplary embodiment may be used as cosmetics.

The cellulose particles according to the exemplary embodiment may be used as cosmetic additives among cosmetics.

Since the cellulose particles according to the exemplary embodiment have high flexibility, the use of the cellulose particles as cosmetic additives tends to improve the spread of cosmetics on the skin when the cosmetics are applied to the skin.

The cellulose particles according to the exemplary embodiment can be used as cosmetic additives for base makeup cosmetics (e.g., makeup bases, concealers, foundations, and face powders); makeup cosmetics (e.g., lipsticks, glosses, lip liners, blushes, eye shadows, eye liners, mascaras, eyebrow pencils, nail polishes, nail care cosmetics); and skin care cosmetics (e.g., face washes, cleansings, lotions, milky lotions, serums, masks, face masks, eye and lip care cosmetics).

Since cosmetic additives for makeup cosmetics require flexibility and biodegradability, the cellulose particles according to the exemplary embodiment may be used as cosmetic additives for makeup cosmetics.

### Examples

Examples will be described below, but the present disclosure is not limited to these Examples. In the following description, the units "part" and "%" are both on a mass basis, unless otherwise specified.

### Preparation of Materials

The following materials are prepared.

### Coating Materials in First Coating Layer

· FT-1: carboxymethyl cellulose (cellulose acylate with acid functional group), "Sunrose MAC 500LC" available from Nippon Paper Industries Co., Ltd.
· FT-2: carboxymethyl cellulose (cellulose acylate with acid functional group), "Daicel 2200" available from Daicel Corporation
· FT-3: carboxymethyl cellulose (cellulose acylate with acid functional group), "Daicel 1160" available from Daicel Corporation
· FT-4: polyacrylic acid (synthetic resin with acid functional group), "JURYMER AC-10SH" available from Toagosei Co., Ltd.
· FT-5: polyacrylic acid (synthetic resin with acid functional group), "JURYMER AC-10H" available from Toagosei Co., Ltd.
· FT-6: xanthan gum (polysaccharide with acid functional group), "Rhaball Gum GS-C" available from Sumitomo Pharma Food & Chemical Co., Ltd.
. FT-7: gellan gum (polysaccharide with acid functional group), "KELCOGEL" available from Sumitomo Pharma Food & Chemical Co., Ltd.
· FT-8: alginate (polysaccharide with acid functional group), "Kimika Algin I-3" available from KIMICA Corporation
· FT-9: alginic acid (polysaccharide with acid functional group), "Alginic Acid" available from Tokyo Chemical Industry Co., Ltd.
· FT-10: carrageenan (polysaccharide with acid functional group), "Sea-Pi Gum FA" available from Sumitomo Pharma Food & Chemical Co., Ltd.
· FT-11: carrageenan (polysaccharide with acid functional group), "κ-Carrageenan" available from FUJIFILM Wako Pure Chemical Corporation.
· FT-12: tamarind gum (neutral polysaccharide), "Glyloid 6C" available from Sumitomo Pharma Food & Chemical Co., Ltd.
· FT-13: dextrin (neutral polysaccharide), "NSD 300A" available from San-ei Sucrochemical Co., Ltd.

### Coating Materials in Second Coating Layer

· ST-1: behenic acid (fatty acid), "NAA-222S" available from NOF Corporation
· ST-2: stearic acid (fatty acid), "NAA-180" available from NOF Corporation
· ST-3: palmitic acid (fatty acid), "Palmitic Acid 98" available from Miyoshi Oil & Fat Co., Ltd.
· ST-4: lauroyl lysine (amino acid compound), "AMIHOPE LL" available from Ajinomoto Co., Inc.
· ST-5: sodium myristoyl glutamate (sodium salt of amino acid compound), "Aminosurfact AMMS-P1" available from Asahi Kasei Corporation
. ST-6: sodium stearoyl glutamate (sodium salt of amino acid compound), "Eumulgin SG" available from BASF

### Examples 1 to 24, Examples 101 to 113, Comparative Examples 1 to 3

### Formation of Base Particles

One hundred thirty parts of DAC " 'L-50' available from Daicel Corporation, cellulose diacetate, weight-average degree of polymerization 570", which is a cellulose acylate, is completely dissolved in 870 parts of ethyl acetate. The resulting solution is added to an aqueous liquid containing 45 parts of calcium carbonate and 500 parts of pure water, and the mixture is stirred for 3 hours. To the mixture, a solution of 4 parts of carboxymethyl cellulose (hereinafter also referred to as "CMC") and 200 parts of methyl ethyl ketone dispersed in 600 parts of pure water is added, and the mixture is stirred with a high-speed emulsifier for 5 minutes. To the mixture, 10 parts of sodium hydroxide is added. The resulting mixture is heated to 80°C under stirring for 3 hours to remove ethyl acetate and methyl ethyl ketone. To the mixture, the same amount of diluted hydrochloric acid as sodium hydroxide is added, and the residue is filtered out and dispersed again in pure water to produce a slurry (solid concentration 10%) of cellulose acylate particles.

To 500 parts of the slurry of cellulose acylate particles, 17.5 parts of a 20% aqueous solution of sodium hydroxide is added, and the mixture is stirred at a saponification temperature of 30°C for 6 hours. After saponification, the pH of the slurry is adjusted to 7 by adding hydrochloric acid to the slurry. The slurry is then filtered and washed repeatedly until the conductivity of the filtrate reaches 10 µS/cm or less to produce a slurry of cellulose base particles.

### Surface Treatment

### Formation of First Coating Layer

According to Table 1, the base particles are subjected to any one of the following cross-linking treatments A, C, and M.

### Cross-Linking Treatment A

The coating material of the type and amount shown in Table 1 is added to 500 parts by mass of the slurry (solid content: 10 mass%) of the base particles. If the slurry of the base particles has pH 6.5 or lower, the slurry of the base particles is neutralized to pH 7.0 to pH 8.5 by adding an aqueous solution of sodium hydroxide to the slurry of the base particles. To the slurry of the base particles, an aqueous solution of potash alum is added over 2 hours such that the amount of potassium alum is 15 mass% with respect to the coating material, and the mixture is stirred for several hours to 24 hours. This process forms, on the surfaces of the base particles, a first coating layer having the coating weight shown in Table 1 and composed of an Al salt of the compound shown in Table 1.

### Cross-Linking Treatment C

The coating material of the type and amount shown in Table 1 is added to 500 parts by mass of the slurry (solid content: 10 mass%) of the base particles. If the slurry of the base particles has pH 6.5 or lower, the slurry of the base particles is neutralized to pH 7.0 to pH 8.5 by adding an aqueous solution of sodium hydroxide to the slurry of the base particles. To the slurry of the base particles, a 0.01 M aqueous solution of calcium chloride is added over 2 hours such that the molar amount of calcium chloride is the same as that of the acid functional group of the coating material, and the mixture is stirred for several hours to 24 hours. This process forms, on the surfaces of the base particles, a first coating layer having the coating weight shown in Table 1 and composed of a Ca salt of the compound shown in Table 1.

### Cross-Linking Treatment M

The coating material of the type and amount shown in Table 1 is added to 500 parts by mass of the slurry (solid content: 10 mass%) of the base particles. If the slurry of the base particles has pH 6.5 or lower, the slurry of the base particles is neutralized to pH 7.0 to pH 8.5 by adding an aqueous solution of sodium hydroxide to the slurry of the base particles. To the slurry of the base particles, a 0.01 M aqueous solution of magnesium chloride is added over 2 hours such that the molar amount of magnesium chloride is the same as that of the acid functional group of the coating material, and the mixture is stirred for several hours to 24 hours. This process forms, on the surfaces of the base particles, a first coating layer having the coating weight shown in Table 1 and composed of a Mg salt of the coating material shown in Table 1. Formation of Second Coating Layer

According to Table 1, the base particles each having the first coating layer thereon are subjected to any one of the following deposition treatments H, A, C, and M.

### Deposition Treatment H

Next, the slurry of the base particles each having the first coating layer thereon is heated to 70°C, and the coating material of the type and amount shown in Table 1 is added to the slurry of the base particles. When the coating material is a fatty acid or an amino acid compound, the fatty acid is neutralized and dissolved by adding an aqueous solution of sodium hydroxide in an amount sufficient to neutralize 95% of the fatty acid. After dissolution of the coating material is confirmed, the slurry of the base particles is stirred for 5 minutes, and the pH of the slurry of the base particles is adjusted to 2 by adding 0.1 M nitric acid to the slurry of the base particles over 20 minutes. After completion of addition, the slurry of the base particles is stirred for 5 minutes, and 1,000 parts by mass of ion exchange water at 0°C to 10°C is then added to the slurry being stirred. This process forms, on the surfaces of the base particles each having the first coating layer thereon, a second coating layer having the coating weight shown in Table 1 and composed of the coating material (non-metal salt coating material) shown in Table 1.

### Deposition Treatment A

Next, the slurry of the base particles each having the first coating layer thereon is heated to 70°C, and the coating material of the type and amount shown in Table 1 is added to the slurry of the base particles. When the coating material is a fatty acid or an amino acid compound, the fatty acid is neutralized and dissolved by adding an aqueous solution of sodium hydroxide in an amount sufficient to neutralize 95% of the fatty acid. After dissolution of the coating material is confirmed, the slurry of the base particles is stirred for 5 minutes, and a 0.1 M aqueous solution of aluminum chloride is added to the slurry of the base particles over 20 minutes such that the molar amount of aluminum chloride (hexahydrate) is the same as that of the coating material. After completion of addition, the slurry of the base particles is stirred for 5 minutes, and 1,000 parts by mass of ion exchange water at 0°C to 10°C is then added to the slurry being stirred. This process forms, on the surfaces of the base particles each having the first coating layer thereon, a second coating layer having the coating weight shown in Table 1 and composed of an Al salt of the coating material shown in Table 1.

### Deposition Treatment C

Next, the slurry of the base particles each having the first coating layer thereon is heated to 70°C, and the coating material of the type and amount shown in Table 1 is added to the slurry of the base particles. When the coating material is a fatty acid or an amino acid compound, the fatty acid is neutralized and dissolved by adding an aqueous solution of sodium hydroxide in an amount sufficient to neutralize 95% of the fatty acid. After dissolution of the coating material is confirmed, the slurry of the base particles is stirred for 5 minutes, and a 0.1 M aqueous solution of calcium chloride is added to the slurry of the base particles over 20 minutes such that the molar amount of calcium chloride is the same as that of the coating material. After completion of addition, the slurry of the base particles is stirred for 5 minutes, and 1,000 parts by mass of ion exchange water at 0°C to 10°C is then added to the slurry being stirred. This process forms, on the surfaces of the base particles each having the first coating layer thereon, a second coating layer having the coating weight shown in Table 1 and composed of a Ca salt of the coating material shown in Table 1.

### Deposition Treatment M

Next, the slurry of the base particles each having the first coating layer thereon is heated to 70°C, and the coating material of the type and amount shown in Table 1 is added to the slurry of the base particles. When the coating material is a fatty acid or an amino acid compound, the fatty acid is neutralized and dissolved by adding an aqueous solution of sodium hydroxide in an amount sufficient to neutralize 95% of the fatty acid. After dissolution of the coating material is confirmed, the slurry of the base particles is stirred for 5 minutes, and a 0.1 M aqueous solution of magnesium chloride is added to the slurry of the base particles over 20 minutes such that the molar amount of magnesium chloride is the same as that of the coating material. After completion of addition, the slurry of the base particles is stirred for 5 minutes, and 1,000 parts by mass of ion exchange water at 0°C to 10°C is then added to the slurry being stirred. This process forms, on the surfaces of the base particles each having the first coating layer thereon, a second coating layer having the coating weight shown in Table 1 and composed of a Mg salt of the coating material shown in Table 1.

### Washing Treatment

The slurry of the base particles having the first coating layer and the second coating layer formed thereon is filtered, and the filtered-out material is washed with pure water and filtered again. This process is repeated until the conductivity of the filtrate reaches 50 µS/cm or less, and the filtered-out material is then freeze-dried to form cellulose particles.

The cellulose particles are stirred in an FM mixer (FM 40, available from Nippon Coke & Engineering Co., Ltd.) at a number of rotation of 2000 min⁻¹ for 3 hours while the temperature of the mixer is maintained at 25°C, whereby the surface of the coating layer is made smooth.

In Comparative Example 1, stirring is performed for 24 hours without any cross-linking treatment during formation of the first coating layer, whereby a first coating layer made of a non-metal salt coating material is formed on the surfaces of the base particles to produce cellulose particles.

### Evaluation of Physical Properties

### Volume-Average Particle Diameter

The volume-average particle diameter ("D50v" in Table) of the cellulose particles produced in Examples is measured in accordance with the method described above. Evaluation

The biodegradation rate, the initial hydrophobicity, the time-dependent hydrophobicity of the cellulose particles produced in Examples are evaluated as described below. Biodegradation Rate

The biodegradation rate after 528 days for the cellulose particles produced in Examples is measured and calculated in accordance with JIS K6950: 2000 (ISO 14851: 1999).

### Initial Hydrophobicity

The cellulose particles (50 mg) produced in Examples are placed in a glass vial containing 5 g of water. The glass vial is closed with a cap and shaked to mix. The glass vial is allowed to stand, and the particles are evaluated based on whether the particles float or settle.

Specifically, the particles are graded and evaluated on a five level scale from G1 to G5, where G5 indicates that the particles are completely floating up, and G1 indicates that the particles are completely settling down.

### Time-Dependent Hydrophobicity

A 0.01% aqueous solution of CETETH 15 (surfactant) is prepared. To 10 g of the aqueous solution, 0.1 g of the cellulose particles produced in Examples are added and immersed therein at 25°C for 7 days. After immersion, the particles are filtered out, washed, and dried. The dried particles are evaluated in the same manner as for the initial hydrophobicity.

**[Table 1-1]**

| | First Coating Layer | | | | | Second Coating Layer | | | Particle Characteristic | Evaluation | | |
|---|---|---|---|---|---|---|---|---|---|---|---|---|
| | coating material_1 | | coating material_2 | | cross-linking treatment | coating material | | deposition treatment | D50v (µm) | biodegradation rate % | initial hydrophobicity | time-dependent hydrophobicity |
| | Type | amount (parts) | Type | amount (parts) | | Type | amount (parts) | | | | | |
| Example 1 | FT-1 | 0.25 | | | KA | ST-1 | 4 | H | 8 | 77 | G3 | G3 |
| Example 2 | FT-2 | 0.25 | | | KA | ST-1 | 4 | C | 7 | 77 | G4 | G3 |
| Example 3 | FT-3 | 0.25 | | | KA | ST-1 | 4 | C | 7 | 77 | G4 | G3 |
| Example 4 | FT-2 | 0.1 | FT-4 | 0.1 | KA | ST-1 | 4 | C | 8 | 77 | G4 | G3 |
| Example 5 | FT-4 | 0.25 | | | C | ST-2 | 4 | C | 6 | 73 | G5 | G4 |
| Example 6 | FT-5 | 0.25 | | | C | ST-2 | 4 | C | 7 | 73 | G5 | G4 |
| Example 7 | FT-5 | 0.25 | | | M | ST-2 | 4 | C | 7 | 73 | G5 | G3 |
| Example 8 | FT-6 | 0.25 | | | KA | ST-2 | 4 | H | 8 | 78 | G3 | G3 |
| Example 9 | FT-6 | 0.25 | | | C | ST-2 | 4 | C | 8 | 78 | G5 | G4 |
| Example 10 | FT-6 | 0.25 | | | C | ST-2 | 4 | M | 7 | 75 | G5 | G3 |
| Example 11 | FT-6 | 0.25 | | | C | ST-2 | 4 | A | 7 | 75 | G5 | G4 |
| Example 12 | FT-6 | 0.25 | | | C | ST-3 | 4 | C | 7 | 78 | G5 | G4 |
| Example 13 | FT-6 | 0.25 | | | C | ST-5 | 4 | A | 7 | 77 | G5 | G4 |
| Example 14 | FT-6 | 0.25 | | | C | ST-6 | 4 | A | 8 | 76 | G5 | G4 |
| Example 15 | FT-6 | 0.15 | FT-7 | 0.1 | C | ST-2 | 4 | C | 7 | 78 | G5 | G4 |
| Example 16 | FT-6 | 0.15 | FT-8 | 0.1 | C | ST-2 | 4 | C | 7 | 78 | G5 | G4 |
| Example 17 | FT-7 | 0.25 | | | C | ST-2 | 4 | C | 8 | 78 | G5 | G4 |
| Example 18 | FT-8 | 0.25 | | | C | ST-2 | 4 | C | 6 | 78 | G5 | G4 |
| Example 19 | FT-8 | 0.25 | | | C | ST-4 | 4 | A | 7 | 77 | G5 | G4 |
| Example 20 | FT-9 | 0.25 | | | C | ST-2 | 4 | C | 7 | 78 | G5 | G4 |
| Example 21 | FT-10 | 0.25 | | | C | ST-2 | 4 | H | 8 | 78 | G3 | G4 |
| Example 22 | FT-10 | 0.25 | | | C | ST-2 | 4 | C | 8 | 78 | G5 | G4 |
| Example 23 | FT-10 | 0.25 | | | C | ST-6 | 4 | A | 6 | 77 | G5 | G4 |
| Example 24 | FT-11 | 0.25 | | | C | ST-2 | 4 | C | 7 | 78 | G5 | G4 |
| Comparative Example 1 | FT-1 | 0.25 | | | no treatment | ST-1 | 4 | C | 7 | 78 | G3 | G1 |
| Comparative Example 2 | FT-12 | 0.25 | | | C | ST-2 | 4 | C | 7 | 78 | G4 | G1 |
| Comparative Example 3 | FT-13 | 0.25 | | | C | ST-2 | 4 | C | 8 | 78 | G4 | G1 |

**[Table 1-2]**

| | First Coating Layer | | | | Second Coating Layer | | | | |
|---|---|---|---|---|---|---|---|---|---|
| | coating material_1 | | molar ratio of multivalent metal to resin | cross-linking treatment | coating material | | deposition treatment | initial hydrophobicity | time-dependent hydrophobicity |
| | Type | amount (parts) | | | Type | amount (parts) | | | |
| Example 101 | FT-6 | 0.4 | 1/3 | C | ST-6 | 5 | C | G3 | G3 |
| Example 102 | FT-6 | 0.4 | 1 | C | ST-6 | 5 | C | G4 | G4 |
| Example 103 | FT-6 | 0.4 | 2 | C | ST-6 | 5 | C | G4 | G4 |
| Example 104 | FT-6 | 0.4 | 3 | C | ST-6 | 5 | C | G4 | G4 |
| Example 105 | FT-6 | 0.4 | 1/4 | C | ST-6 | 5 | C | G3 | G2 |
| Example 106 | FT-6 | 0.4 | 4 | C | ST-6 | 5 | C | G3 | G2 |
| Example 107 | FT-8 | 0.4 | 1 | C | ST-6 | 2 | C | G4 | G4 |
| Example 108 | FT-8 | 0.4 | 1 | C | ST-6 | 10 | C | G4 | G4 |
| Example 109 | FT-8 | 0.4 | 1 | C | ST-6 | 15 | C | G4 | G4 |
| Example 110 | FT-8 | 0.4 | 1 | C | ST-6 | 1 | C | G3 | G2 |
| Example 111 | FT-8 | 0.4 | 1 | C | ST-6 | 20 | C | G4 | G3 |
| Example 112 | FT-8 | 0.4 | 1 | C | ST-6 | 0.5 | C | G2 | G2 |
| Example 113 | FT-8 | 0.4 | 1 | C | ST-6 | 30 | C | G4 | G2 |

The above results reveal that the cellulose particles of Examples show more rapid biodegradation, have better water resistance, and keep water resistance even after being stored in water compared with the cellulose particles of Comparative Examples.

The foregoing description of the exemplary embodiments of the present disclosure has been provided for the purposes of illustration and description. It is not intended to be exhaustive or to limit the disclosure to the precise forms disclosed. Obviously, many modifications and variations will be apparent to practitioners skilled in the art. The embodiments were chosen and described in order to best explain the principles of the disclosure and its practical applications, thereby enabling others skilled in the art to understand the disclosure for various embodiments and with the various modifications as are suited to the particular use contemplated. It is intended that the scope of the disclosure be defined by the following claims and their equivalents.

### Appendix

(((1))) A cellulose particle including:
   a base particle containing cellulose as a main component;
   a first coating layer disposed on a surface of the base particle and containing a multivalent metal salt of an acid functional group-containing resin; and
   a second coating layer disposed on the first coating layer and containing at least one hydrophobic compound selected from fatty acids, multivalent metal salts of fatty acids, amino acid compounds, and multivalent metal salts of amino acid compounds.
(((2))) The cellulose particle according to (((1))), wherein the multivalent metal salt of the acid functional group-containing resin is a multivalent metal salt of a polysaccharide.
(((3))) The cellulose particle according to (((1))) or (((2))), wherein the multivalent metal salt of the acid functional group-containing resin has at least one multivalent metal selected from Ca and Al.
(((4))) The cellulose particle according to any one of (((1))) to (((3))), wherein a ratio of a multivalent metal to the resin in the multivalent metal salt of the acid functional group-containing resin is 1/4 or more and 4/1 or less in terms of molar ratio.
(((5))) The cellulose particle according to (((4))), wherein the ratio of the multivalent metal to the resin in the multivalent metal salt of the acid functional group-containing resin is 1/3 or more and 3/1 or less in terms of molar ratio.
(((6))) The cellulose particle according to any one of (((1))) to (((5))), wherein the hydrophobic compound is at least one hydrophobic compound selected from the multivalent metal salts of fatty acids and the multivalent metal salts of amino acid compounds.
(((7))) The cellulose particle according to (((6))), wherein the multivalent metal salts of fatty acids and the multivalent metal salts of amino acid compounds have at least one multivalent metal selected from Ca and Al.
(((8))) The cellulose particle according to any one of (((1))) to (((7))),
   wherein the multivalent metal salt of the acid functional group-containing resin is a multivalent metal salt of a polysaccharide,
   the multivalent metal salt of the acid functional group-containing resin has at least one multivalent metal selected from Ca and Al, and
   a ratio of a multivalent metal to the resin in the multivalent metal salt of the acid functional group-containing resin is 1/3 or more and 3/1 or less in terms of molar ratio.
(((9))) The cellulose particle according to (((8))), wherein the hydrophobic compound is at least one hydrophobic compound selected from the multivalent metal salts of fatty acids and the multivalent metal salts of amino acid compounds.
(((10))) The cellulose particle according to (((9))), wherein the multivalent metal salts of fatty acids and the multivalent metal salts of amino acid compounds have at least one multivalent metal selected from Ca and Al.

The advantageous effects of the aspects are as described below.

According to (((1))), the cellulose particle having the base particle containing cellulose as a main component shows more rapid biodegradation, has better water resistance, and keeps water resistance even after being stored in water compared with a cellulose particle having a first coating layer disposed on the surface of a base particle and containing only a neutral polysaccharide and a second coating layer disposed on the first coating layer and containing a hydrophobic compound.

According to (((2))), the cellulose particle shows more rapid biodegradation, has better water resistance, and keeps water resistance even after being stored in water compared with a particle in which the multivalent metal salt of an acid functional group-containing resin is a multivalent metal salt of a synthetic resin.

According to (((3))), the cellulose particle shows more rapid biodegradation, has better water resistance, and keeps water resistance even after being stored in water compared with a particle in which the multivalent metal salt of an acid functional group-containing resin has a multivalent metal other than Ca and Al.

According to (((4))), the cellulose particle shows more rapid biodegradation, has better water resistance, and keeps water resistance even after being stored in water compared with a particle in which the ratio of the multivalent metal to the resin in the multivalent metal salt of the acid functional group-containing resin is less than 1/4 or more than 4/1 in terms of molar ratio.

According to (((5))), the cellulose particle shows more rapid biodegradation, has better water resistance, and keeps water resistance even after being stored in water compared with a particle in which the ratio of the multivalent metal to the resin in the multivalent metal salt of the acid functional group-containing resin is less than 1/3 or more than 3/1 in terms of molar ratio.

According to (((6))), the cellulose particle shows more rapid biodegradation, has better water resistance, and keeps water resistance even after being stored in water compared with a particle in which the hydrophobic compound is at least one hydrophobic compound selected from fatty acids and amino acid compounds.

According to (((7))), the cellulose particle shows more rapid biodegradation, has better water resistance, and keeps water resistance even after being stored in water compared with a particle in which the multivalent metal salts of fatty acids and the multivalent metal salts of amino acid compounds have a multivalent metal other than Ca and Al.

According to (((8))), the cellulose particle shows more rapid biodegradation, has better water resistance, and keeps water resistance even after being stored in water compared with a particle in which the multivalent metal salt of an acid functional group-containing resin is a multivalent metal salt of a synthetic resin, the multivalent metal salt of an acid functional group-containing resin has a multivalent metal other than Ca and Al, or the ratio of the multivalent metal to the resin in the multivalent metal salt of the acid functional group-containing resin is less than 1/4 or more than 4/1 in terms of molar ratio.

According to (((9))), the cellulose particle shows more rapid biodegradation, has better water resistance, and keeps water resistance even after being stored in water compared with a particle in which the hydrophobic compound is at least one hydrophobic compound selected from fatty acids and amino acid compounds.

According to (((10))), the cellulose particle shows more rapid biodegradation, has better water resistance, and keeps water resistance even after being stored in water compared with a particle in which the multivalent metal salts of fatty acids and the multivalent metal salts of amino acid compounds have a multivalent metal other than Ca and Al.

## Claims

1. A cellulose particle comprising:
a base particle containing cellulose as a main component;
a first coating layer disposed on a surface of the base particle and containing a multivalent metal salt of an acid functional group-containing resin; and
a second coating layer disposed on the first coating layer and containing at least one hydrophobic compound selected from fatty acids, multivalent metal salts of fatty acids, amino acid compounds, and multivalent metal salts of amino acid compounds.

2. The cellulose particle according to claim 1, wherein the multivalent metal salt of the acid functional group-containing resin is a multivalent metal salt of a polysaccharide.

3. The cellulose particle according to claim 1 or 2, wherein the multivalent metal salt of the acid functional group-containing resin has at least one multivalent metal selected from Ca and Al.

4. The cellulose particle according to any one of claims 1 to 3, wherein a ratio of the multivalent metal to the resin in the multivalent metal salt of the acid functional group-containing resin is 1/4 or more and 4/1 or less in terms of molar ratio.

5. The cellulose particle according to claim 4, wherein the ratio of the multivalent metal to the resin in the multivalent metal salt of the acid functional group-containing resin is 1/3 or more and 3/1 or less in terms of molar ratio.

6. The cellulose particle according to any one of claims 1 to 5, wherein the hydrophobic compound is at least one hydrophobic compound selected from the multivalent metal salts of fatty acids and the multivalent metal salts of amino acid compounds.

7. The cellulose particle according to claim 6, wherein the multivalent metal salts of fatty acids and the multivalent metal salts of amino acid compounds have at least one multivalent metal selected from Ca and Al.

8. The cellulose particle according to any one of claims 1 to 7,
wherein the multivalent metal salt of the acid functional group-containing resin is a multivalent metal salt of a polysaccharide,
the multivalent metal salt of the acid functional group-containing resin has at least one multivalent metal selected from Ca and Al, and
a ratio of a multivalent metal to the resin in the multivalent metal salt of the acid functional group-containing resin is 1/3 or more and 3/1 or less in terms of molar ratio.

9. The cellulose particle according to claim 8, wherein the hydrophobic compound is at least one hydrophobic compound selected from the multivalent metal salts of fatty acids and the multivalent metal salts of amino acid compounds.

10. The cellulose particle according to claim 9, wherein the multivalent metal salts of fatty acids and the multivalent metal salts of amino acid compounds have at least one multivalent metal selected from Ca and Al.

## Patentansprüche

1. Zellulosepartikel, umfassend:
ein Basispartikel, das Zellulose als eine Hauptkomponente enthält;
eine erste Beschichtungsschicht, die auf einer Oberfläche des Basispartikels angeordnet ist und ein multivalentes Metallsalz eines Harzes, das Funktionsgruppe von Säure enthält, enthält; und
eine zweite Beschichtungsschicht, die auf der ersten Beschichtungsschicht angeordnet ist und mindestens eine hydrophobe Verbindung, die aus Fettsäuren, multivalenten Metallsalzen von Fettsäuren, Aminosäureverbindungen und multivalenten Metallsalzen von Aminosäureverbindungen ausgewählt wird, enthält.

2. Zellulosepartikel nach Anspruch 1, wobei das multivalente Metallsalz des Harzes, das Funktionsgruppe von Säure enthält, ein multivalentes Metallsalz eines Polysaccharids ist.

3. Zellulosepartikel nach Anspruch 1 oder 2, wobei das multivalente Metallsalz des Harzes, das Funktionsgruppe von Säure enthält, mindestens ein multivalentes Metall, das aus Ca und Al ausgewählt wird, aufweist.

4. Zellulosepartikel nach einem der Ansprüche 1 bis 3, wobei ein Verhältnis des multivalenten Metalls zu dem Harz bei dem multivalenten Metallsalz des Harzes, das Funktionsgruppe von Säure enthält, 1/4 oder mehr und 4/1 oder weniger in Bezug auf Molverhältnis beträgt.

5. Zellulosepartikel nach Anspruch 4, wobei das Verhältnis des multivalenten Metalls zu dem Harz bei dem multivalenten Metallsalz des Harzes, das Funktionsgruppe von Säure enthält, 1/3 oder mehr und 3/1 oder weniger in Bezug auf Molverhältnis beträgt.

6. Zellulosepartikel nach einem der Ansprüche 1 bis 5, wobei die hydrophobe Verbindung mindestens eine hydrophobe Verbindung ist, die aus den multivalenten Metallsalzen von Fettsäuren und den multivalenten Metallsalzen von Aminosäureverbindungen ausgewählt wird.

7. Zellulosepartikel nach Anspruch 6, wobei die multivalenten Metallsalze von Fettsäuren und die multivalenten Metallsalze von Aminosäureverbindungen mindestens ein multivalentes Metall, das aus Ca und Al ausgewählt wird, aufweisen.

8. Zellulosepartikel nach einem der Ansprüche 1 bis 7,
wobei das multivalente Metallsalz des Harzes, das Funktionsgruppe von Säure enthält, ein multivalentes Metallsalz eines Polysaccharids ist,
das multivalente Metallsalz des Harzes, das Funktionsgruppe von Säure enthält, mindestens ein multivalentes Metall, das aus Ca und Al ausgewählt wird, aufweist, und
ein Verhältnis eines multivalenten Metalls zu dem Harz bei dem multivalenten Metallsalz des Harzes, das Funktionsgruppe von Säure enthält, 1/3 oder mehr und 3/1 oder weniger in Bezug auf Molverhältnis beträgt.

9. Zellulosepartikel nach Anspruch 8, wobei die hydrophobe Verbindung mindestens eine hydrophobe Verbindung ist, die aus den multivalenten Metallsalzen von Fettsäuren und den multivalenten Metallsalzen von Aminosäureverbindungen ausgewählt wird.

10. Zellulosepartikel nach Anspruch 9, wobei die multivalenten Metallsalze von Fettsäuren und die multivalenten Metallsalze von Aminosäureverbindungen mindestens ein multivalentes Metall, das aus Ca und Al ausgewählt wird, aufweisen.

## Revendications

1. Particule de cellulose comprenant :
une particule de base contenant de la cellulose comme composant principal ;
une première couche de revêtement disposée sur une surface de la particule de base et contenant un sel de métal multivalent d'une résine contenant un groupe fonctionnel acide ; et
une deuxième couche de revêtement disposée sur la première couche de revêtement et contenant au moins un composé hydrophobe choisi parmi des acides gras, des sels de métal multivalent d'acides gras, des composés d'acides aminés et des sels de métal multivalent de composés d'acides aminés.

2. Particule de cellulose selon la revendication 1, dans laquelle le sel de métal multivalent de la résine contenant un groupe fonctionnel acide est un sel de métal multivalent d'un polysaccharide.

3. Particule de cellulose selon la revendication 1 ou la revendication 2, dans laquelle le sel de métal multivalent de la résine contenant un groupe fonctionnel acide comporte au moins un métal multivalent choisi parmi Ca et Al.

4. Particule de cellulose selon l'une quelconque des revendications 1 à 3, dans laquelle un rapport du métal multivalent à la résine dans le sel de métal multivalent de la résine contenant un groupe fonctionnel acide est de 1/4 ou plus et de 4/1 ou moins en termes de rapport molaire.

5. Particule de cellulose selon la revendication 4, dans laquelle le rapport du métal multivalent à la résine dans le sel de métal multivalent de la résine contenant un groupe fonctionnel acide est de 1/3 ou plus et de 3/1 ou moins en termes de rapport molaire.

6. Particule de cellulose selon l'une quelconque des revendications 1 à 5, dans laquelle le composé hydrophobe est au moins un composé hydrophobe choisi parmi les sels de métal multivalent d'acides gras et les sels de métal multivalent de composés d'acides aminés.

7. Particule de cellulose selon la revendication 6, dans laquelle les sels de métal multivalent d'acides gras et les sels de métal multivalent de composés d'acides aminés comportent au moins un métal multivalent choisi parmi Ca et Al.

8. Particule de cellulose selon l'une quelconque des revendications 1 à 7,
dans lequel le sel de métal multivalent de la résine contenant un groupe fonctionnel acide est un sel de métal multivalent d'un polysaccharide,
le sel de métal multivalent de la résine contenant un groupe fonctionnel acide comporte au moins un métal multivalent choisi parmi Ca et Al, et
un rapport d'un métal multivalent à la résine dans le sel de métal multivalent de la résine contenant un groupe fonctionnel acide est de 1/3 ou plus et de 3/1 ou moins en termes de rapport molaire.

9. Particule de cellulose selon la revendication 8, dans laquelle le composé hydrophobe est au moins un composé hydrophobe choisi parmi les sels de métal multivalent d'acides gras et les sels de métal multivalent de composés d'acides aminés.

10. Particule de cellulose selon la revendication 9, dans laquelle les sels de métal multivalent d'acides gras et les sels de métal multivalent de composés d'acides aminés comportent au moins un métal multivalent choisi parmi Ca et Al.
